(19) Europäisches Patentamt
European Patent Office
Office européen des brevets

(11) Veröffentlichungsnummer: **0 226 072 B1**

# (12) EUROPÄISCHE PATENTSCHRIFT

(45) Veröffentlichungstag der Patentschrift: **10.04.91**

(51) Int. Cl.⁵: **A61K 7/13**, C07C 215/76, C07D 263/22

(21) Anmeldenummer: **86116230.3**

(22) Anmeldetag: **22.11.86**

## (54) Oxidationshaarfärbemittel auf der Baiss von 4-Amino-2-aminomethyl-phenolen.

(30) Priorität: **07.12.85 DE 3543345**

(43) Veröffentlichungstag der Anmeldung:
**24.06.87 Patentblatt 87/26**

(45) Bekanntmachung des Hinweises auf die Patenterteilung:
**10.04.91 Patentblatt 91/15**

(84) Benannte Vertragsstaaten:
**AT BE CH DE ES FR GB GR IT LI LU NL SE**

(56) Entgegenhaltungen:
**EP-A- 0 172 139**
**EP-A- 0 182 187**
**GB-A- 2 018 836**
**US-A- 2 843 585**
**US-A- 3 762 922**

**CHEMICAL ABSTRACTS, Band 101, 1984, Seite 534, Zusammenfassung Nr. 63594w, Columbus Ohio, US**

(73) Patentinhaber: **Wella Aktiengesellschaft**
**Berliner Allee 65**
**W-6100 Darmstadt(DE)**

(72) Erfinder: **Clausen, Thomas, Dr.**
**Ernst-Pasqué-Strasse 35 A**
**W-6146 Alsbach(DE)**
Erfinder: **Konrad, Eugen**
**Mecklenburger Strasse 101**
**W-6100 Darmstadt(DE)**

**EP 0 226 072 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Mittel zur oxidativen Färbung von Haaren auf der Basis von 4-Amino-2-aminomethyl-phenolen als Entwicklersubstanz sowie neue 4-Amino-2-aminomethyl-phenole.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diamino-toluol, 2,5-Diamino-benzylalkohol, p-Aminophenol und 1,4-Diamino-benzol eingesetzt. Von den vorzugsweise verwendeten Kupplersubstanzen sind Resorcin, 4-Chlorresorcin, 1-Naphthol, m-Aminophenol und Derivate des m-Phenylendiamins zu nennen.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch eine Kombination von geeigneten Entwickler- und Kupplersubstanzen eine breite Palette verschiedener Farbnuancen er zeugt werden kann. Zur Erzielung natürlicher und besonders modischer Nuancen im Rotbereich wird vor allem p-Aminophenol, allein oder im Gemisch mit anderen Entwicklersubstanzen, in Kombination mit geeigneten Kupplersubstanzen eingesetzt.

Gegen den für den Rotbereich der Farbskala bisher hauptsächlich eingesetzten Entwickler p-Aminophenol werden in letzter Zeit Bedenken in Bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können.

So weisen beispielsweise die in der DE-A 182 187 beschriebenen p-Aminophenolderivate entweder nicht ausreichende physiologische Eigenschaften auf oder sind präparativ schlecht zugänglich. Auch kann die mit den dort beschriebenen Farbstoffen erzielbare Farbtiefe nicht völlig zufriedenstellen.

Es bestand daher die Aufgabe, Oxidationshaarfärbemittel auf der Basis von Entwicklersubstanzen für den Rotbereich aufzufinden, die den erwähnten Anforderungen besser gerecht werden.

Hierzu wurde nun gefunden, daß durch Mittel zur oxidativen Färbung von Haaren auf der Basis einer Entwicklersubstanz-Kupplersubstanz-Kombination, dadurch gekennzeichnet, daß sie als Entwicklersubstanz ein 4-Amino-2-aminomethyl-phenol der allgemeinen Formel (I)

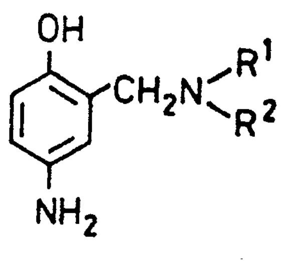 (I),

worin $R^1$ und $R^2$ unabhängig voneinander Wasserstoff, Alkyl, Hydroxyalkyl, Aminoalkyl, Mono- oder Dialkylaminoalkyl, Acylaminoalkyl, Aryl- oder Alkylsul-fonylaminoalkyl, Carbamidoalkyl, Alkylsulfonyl - wobei der Alkylrest jeweils 1 bis 4 Kohlenstoffatome aufweist - Arylsulfonyl, Acyl oder Carbamoyl bedeuten oder durch $R^1$ und $R^2$ ein heterocyclischer,
nicht aromatischer Fünf- oder Sechsring, der zusätzlich eine Oxo-Gruppe aufweisen kann, gebildet wird, oder dessen Salz enthalten, die gestellte Aufgabe in hervorragender Weise gelöst wird.

In den Haarfärbemitteln sollen die erfindungsgemäßen Entwickersubstanzen, von denen das 4-Amino-2-amino-methyl-phenol, das 4-Amino-2-[(2'-hydroxyethyl)-aminomethyl]-phenol und das 4-Amino-2-dimethylamino-methyl-phenol bevorzugt werden, in einer Konzentration von etwa 0,01 bis 3,0 Gewichtsprozent, vorzugsweise 0,1 bis 2,5 Gewichtsprozent, enthalten sein.

Obwohl die vorteilhaften Eigenschaften der hier beschriebenen Entwicklersubstanzen es nahelegen, diese als alleinige Entwickler zu verwenden, ist es selbstverständlich auch möglich, diese Entwicklersubstanzen der Formel (I) gemeinsam mit bekannten Entwicklersubstanzen, wie zum Beispiel 1,4-Diaminobenzol, 2,5-Diamino-toluol, 2,5-Diamino-benzyl-alkohol oder 2,5-Diamino-phenylethylalkohol, einzusetzen.

Von den bekannten Kupplersubstanzen kommen als Bestandteil der hier beschriebenen Haarfärbemittel

vor allem Resorcin, 4-Chlor-resorcin, 4,6-Dichlor-resorcin, 2-Methyl-resorcin, 2-Amino-4-[(2'-hydroxy-ethyl)-amino]-anisol, 2,4-Diamino-benzylalkohol, 2,4-Diamino-phenylethylalkohol, m-Phenylendiamin, 5-Amino-2-methyl-phenol, 2,4-Diamino-phenoxyethanol, 4-Amino-2-hydroxy-phenoxyethanol, 1-Naphthol, m-Aminophenol, 3-Amino-2-methyl-phenol, m-Toluylen-diamin, 4-Hydroxy-1,2-methylendioxy-benzol, 4-Amino-1,2-methylendioxy-benzol, 4-[(2'-Hy-droxyethyl)-amino]-1,2-methylendioxy-benzol, 2,4-Diamino-anisol, 2,4-Diamino-phenetol, 4-Hydroxy-indol, 3-Amino-5-hydroxy-2,6-dimethoxy-pyridin und 3,5-Diamino-2,6-dimethoxy-pyridin in Betracht.

Die genannten Kuppler- und Entwicklersubstanzen können in den Haarfärbemitteln jeweils einzeln oder im Gemisch miteinander enthalten sein.

Die Gesamtmenge der in den hier beschriebenen Haarfärbemitteln enthaltenen Entwicklersubstanz-Kupplersubstanz-Kombination soll etwa 0,1 bis 5,0 Gewichtsprozent, vorzugsweise 0,5 bis 4,0 Gewichtsprozent, betragen.

Die Entwicklersubstanzen werden im allgemeinen in etwa äquimolaren Mengen, bezogen auf die Kupplersubstanzen, eingesetzt. Es ist jedoch nicht nachteilig, wenn die Entwicklersubstanz diesbezüglich in einem gewissen Überschuß oder Unterschuß vorhanden ist.

Weiterhin können die Haarfärbemittel dieser Anmeldung zusätzlich andere Farbkomponenten, beispielsweise 6-Amino-2-methyl-phenol und 2-Amino-5-methyl-phenol, sowie ferner übliche direktziehende Farbstoffe, zum Beispiel Triphenylmethanfarbstoffe, wie beispielsweise Diamond Fuchsine (C.I. 42 510) und Leather Ruby HF (C.I. 42 520), aromatische Nitrofarbstoffe, wie zum Beispiel 2-Nitro-1,4-diamino-benzol, 2-Amino-4-nitro-phenol, 2-Amino-5-nitro-phenol, 2-Amino-4,6-dinitro-phenol, 2-Amino-5-[(2'-hydroxyethyl)-amino]-nitrobenzol und 2-Methyl-amino-5[bis-(2'-hydroxyethyl)-amino]-nitrobenzol, Azofarbstoffe, wie zum Beispiel Acid Brown 4 (C.I. 14 805) und Dispersionsfarbstoffe, wie beispielsweise 1,4-Diamino-anthrachinon und 1,4,5,8-Tetraamino-anthrachinon, enthalten. Die Haarfärbe mittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid oder Sulfat, oder - sofern sie aromatische OH-Gruppen besitzen - in Form der Salze mit Basen, zum Beispiel als Alkaliphenolate, eingesetzt werden.

Darüber hinaus können in den Haarfärbemitteln noch weitere übliche kosmetische Zusätze, beispielsweise Antioxidantien, vozugsweise Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol, sowie mehrwertige Alkohole, wie zum Beispiel Ethylenglykol, Glycerin und 1,2-Propylen-glykol, weiterhin Netzmittel oder Emulgatoren aus den Klassen der anionischen, kationischen, amphoteren oder nichtionogenen oberflächenaktiven Substanzen, wie zum Beispiel Fettalkoholsulfate, Fettalkoholethersulfate, oxethylierte Fettalkoholsulfate, Alkylsulfonate, Alkyl-benzolsulfonate, Alkyltrimethylammoniumsalze, Alkyl-betaine, oxethylierte Fettalkohole, oxethylierte Alkyl-phenole, Fettsäurealkanolamide oder oxethylierte Fettsäureester; ferner Verdicker, wie beispielsweise höhere Fettalkohole, Bentonit, Stärke, Cellulosederivate wie zum Beispiel Carboxymethylcellulose, Alginate, Vaseline, Paraffinöl und Fettsäuren, sowie außerdem Pflegestoffe, wie zum Beispiel kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen, wie beispielsweise Natriumhydroxid und Kaliumhydroxid, Verwendung finden.

Für die Anwendung zur oxidativen Färbung von Haaren vermischt man die vorstehend beschriebenen Haarfärbemittel unmittelbar vor dem Gebrauch mit einem Oxidationsmittel und trägt eine für die Haarfärbebehandlung ausreichende Menge, je nach Haarfülle im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3 bis 12-prozentigen, vorzugsweise 6-prozentigen, wäßrigen Lösung in Betracht. Wird eine 6-prozentige Wasserstoffperoxidlösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 °C etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die Herstellung der Verbindungen der Formel (I) kann auf den im Folgenden beschriebenen drei Wegen A, B und C erfolgen.

A. Durch elektrophile Substitution von p-Nitrophenol (II) mit verschiedenen Elektrophilen nach Schema 1, 2 oder 3, gegebenenfalls weitere Umsetzung in der Seitenkette, und anschließende Reduktion:

Schema 1:

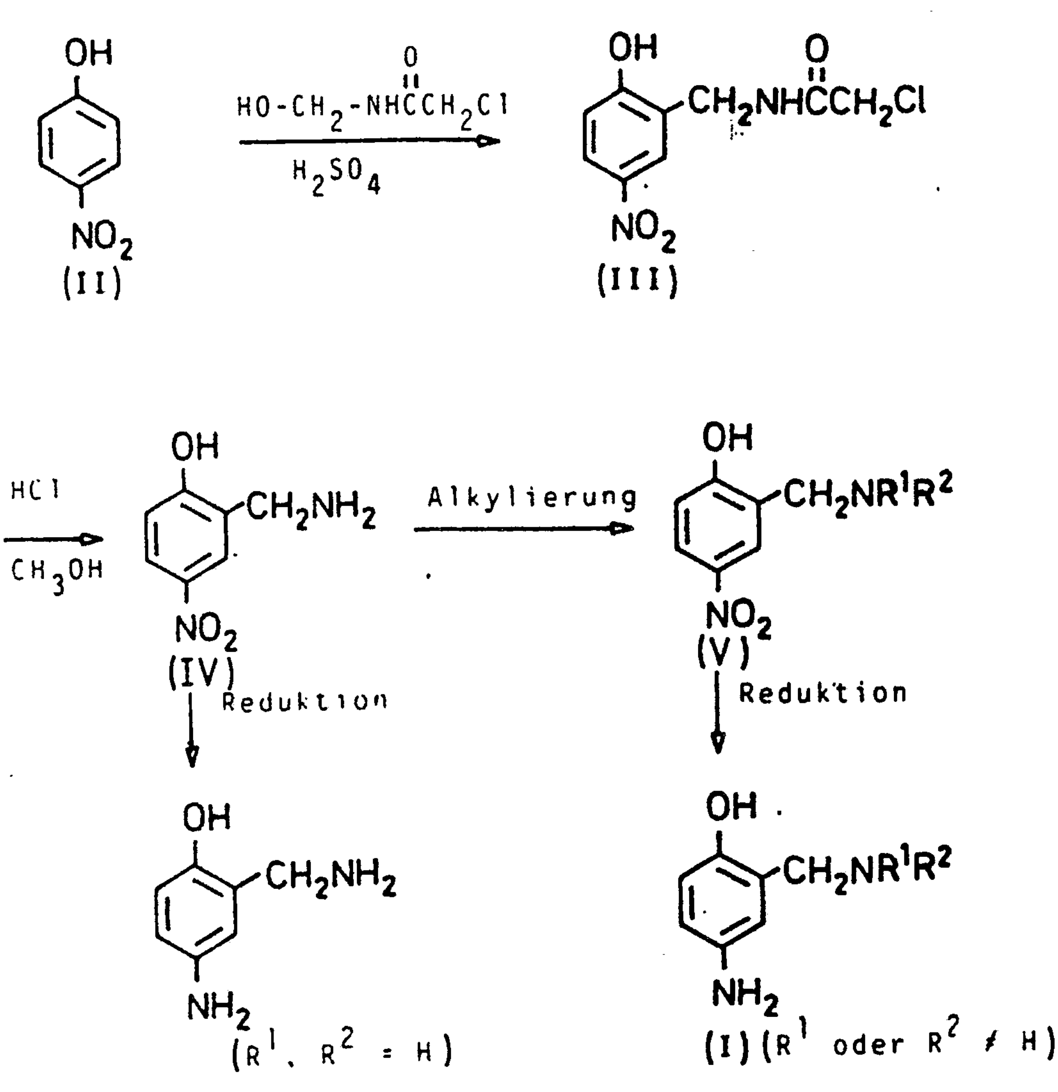

Die Herstellung der Verbindung (III) ist in der Literatur beschrieben (H. E. Zaugg, Synthesis 1984 , Seite 85 bis 96) oder kann analog G. E. Stokker et al., J. Med. Chem. 23 , Seite 1414 bis 1427 (1980) und der DE-PS 21 63 908 erfolgen.

Die Verbindung (IV) (durch Spaltung des Amids (III) erhalten) dient als Ausgangsmaterial für eine ganze Palette von in der Seitenkette substituierten Aminomethylphenolen, kann aber auch direkt durch Reduktion in eine Entwicklersubstanz der Formel (I) überführt werden.

Die Reduktion von (IV) oder (V) kann zum Beispiel katalytisch (beispielsweise unter Verwendung eines Platinkatalysators) ausgeführt werden, wobei vorteilhaft auch Hydrazin als Wasserstoffüberträger Verwendung finden kann.

Schema 2:

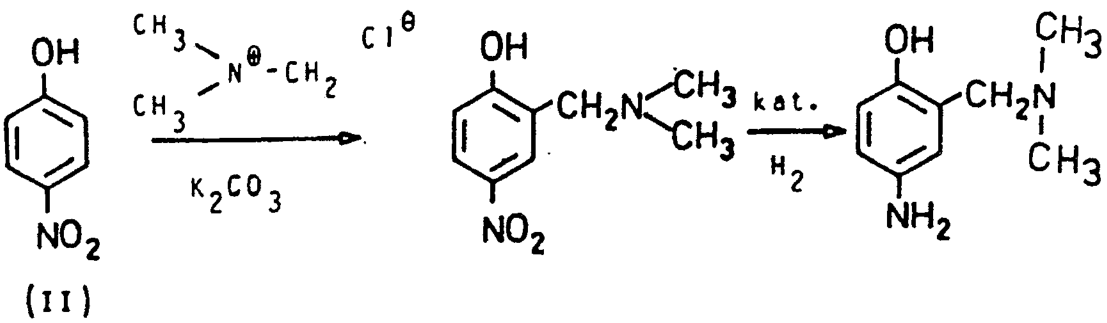


Die Umsetzung des Nitrophenols (II) kann auch analog R. Ungaro et al., Synthesis 1983 , Seite 906 und 907, mit N-Methyl-N-methylen-methanimmoniumchlorid erfolgen. Nach der Reduktion wird direkt die Dimethylverbindung ($R^1$, $R^2$ = $CH_3$) erhalten.

Schema 3:

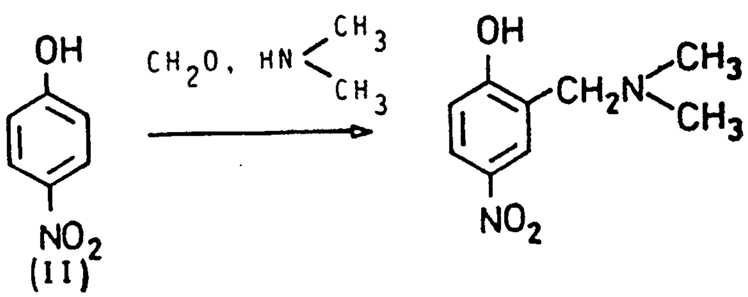

Die Aminomethylierung von (II) kann ferner durch die Mannich-Reaktion (Schema 3) erfolgen, (vergleiche R. Schröter in "Methoden der organischen Chemie" (Houben-Weyl), Bd. XI/I, Seite 731 ff., 4. Auflage, Georg Thieme Verlag, Stuttgart 1957)

B. Durch Substitution des Broms im 2-Hydroxy-5-nitro-benzylbromid (VI) läßt sich mit geeigneten Aminen eine ganze Palette von Vorstufen (V) herstellen. Die Reduktion ergibt jeweils die Entwicklersubstanz der Formel (I).

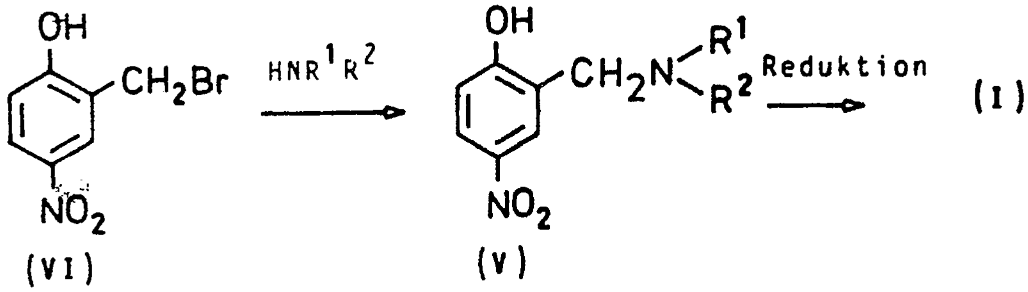


C. Schließlich lassen sich die erfindungsgemäßen Entwicklersubstanzen durch die Umsetzung des 4-Nitro-salicylaldehyds (VII) mit geeigneten Aminen und Reduktion der entstandenen Schiff'schen Basen beziehungsweise Immoniumverbindungen, vorteilhaft zum Beispiel nach den Verfahren von R. F. Borch et al., J. Am. Chem. Soc. 93 , Seite 2897 bis 2904 (1971) oder F. Dallacker et al., Chem. Ber. 104 , Seite 2517 bis 2525 (1971) gewinnen.

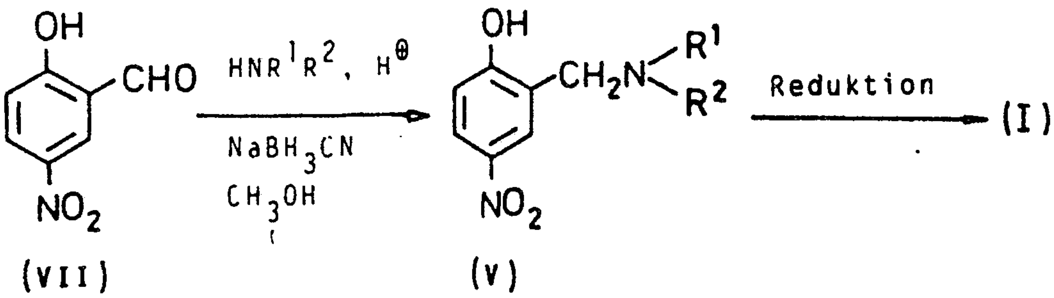


Ein weiterer Syntheseweg verläuft über das Oxazolidinon (VIII). Dieses kann einerseits direkt zu einer Entwicklersubstanz der Formel (I) reduziert werden, andererseits kann es aber auch nach folgendem Schema zur Herstellung weiterer erfindungsgemäßer Verbindungen dienen:

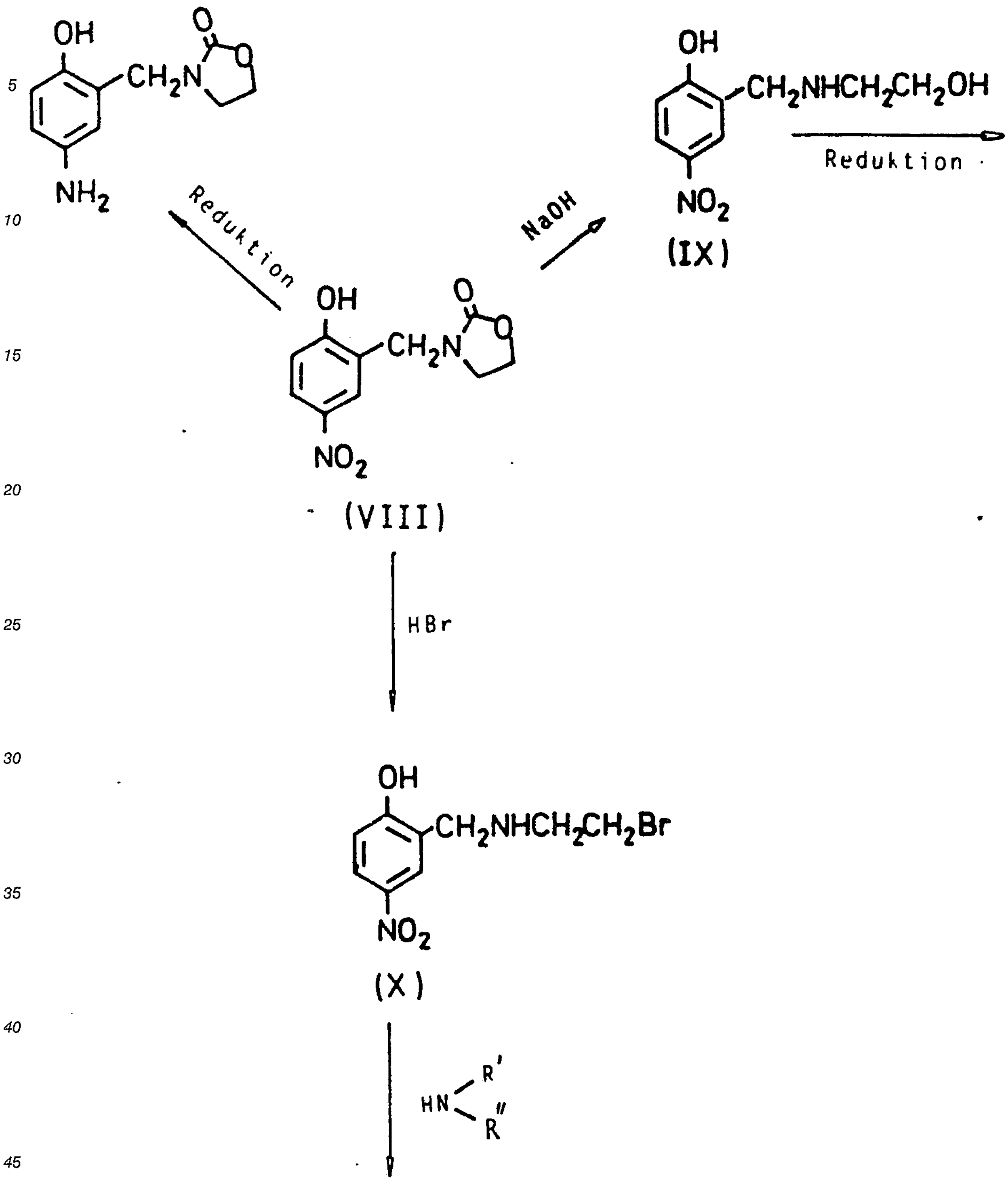


(IX)

(VIII)

(X)

12

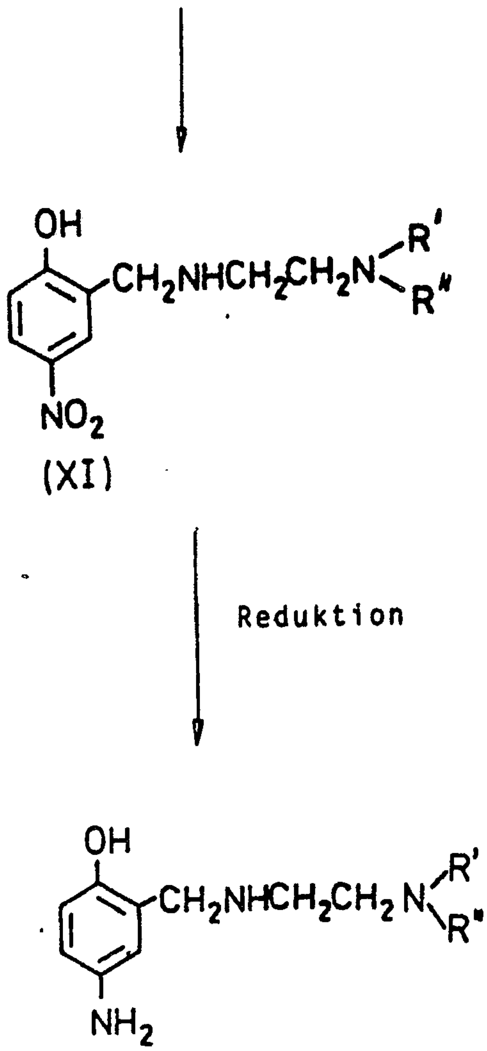


(XI)

Zahlreiche Verbindungen der Formel (I) sind bereits bekannt. Ihre Herstellung ist unter anderem in den nachfolgenden Literaturstellen beschrieben:

- Beilsteins Handbuch der Organischen Chemie (1930) 13 , 598; ibid (1973) 13 , E III 1548; ibid (1985) 13 , E IV 1691 - 1692;
- Angelo et al., J. Med. Chem. (1983) 26 , 1258 - 1267 und 1311 - 1316;
- Burckhalter et al., J. Am. Chem. Soc. (1948) 70 , 1363 - 1373.

Die Entwicklersubstanzen der Formel (I) sind zum Teil neu. Gegenstand der vorliegenden Erfindung sind daher weiterhin neue 4-Amino-2-aminomethyl-phenole der Formel (XII)

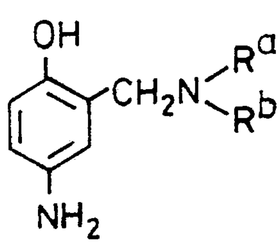

(XII),

worin $R^a$ gleich Wasserstoff, Alkyl, Hydroxyalkyl, Aminoalkyl, Mono- oder Dialkylaminoalkyl, Acylaminoalkyl, Aryl- oder Alkylsulfonylaminoalkyl, Carbamoyl, Carbamidoalkyl, Acyl, Aryl- oder Alkylsulfonyl - wobei die Alkylreste jeweils 1 bis 4 Kohlenstoffatome aufweisen - bedeutet,$R^b$ gleich Aminoalkyl, Mono- oder

Dialkylaminoalkyl, Acylaminoalkyl, Aryl- oder Alkylsulfonylaminoalkyl, Carbamoyl, Carbamidoalkyl, Acyl, Arylsulfonyl-wobei die Alkylreste jeweils 1 bis 4 Kohlenstoffatome aufweisen - ist.

Beispiele für erfindungsgemäße Verbindungen der Formel (XII) sind 2-Acetamidomethyl-4-amino-phenol und N-(5'-Amino-2'-hydroxy-benzyl)-N',N'-diethyl-ethylendiamin.

Die Herstellung dieser neuen Verbindungen erfolgt nach den bereits beschriebenen Herstellungsverfahren A, B und C und wird in den Herstellungsbeispielen näher erläutert.

Die Salze der Verbindungen der Formel (I) und sind durch Umsetzung mit organischen oder anorganischen Säuren oder Basen erhältlich.

In den erfindungsgemäßen Haarfärbemitteln soll die Entwicklersubstanz der Formel (I) und entweder als freie Base oder in Form ihrer physiologisch verträglichen Salze mit anorganischen oder organischen Säuren, zum Beispiel als Chlorid, Sulfat, Phosphat, Acetat, Propionat, Lactat oder Citrat, eingesetzt werden. Im stark alkalischen Medium können sie ferner als Phenolat vorliegen. Die Verbindungen der Formel (I) sind gut in Wasser löslich. Sie weisen außerdem eine ausgezeichnete Lagerstabilität, insbesondere als Bestandteil der hier beschriebenen Haarfärbemittel, auf.

Die erfindungsgemäßen Haarfärbemittel auf der Basis der 4-Amino-2-aminomethyl-phenole als Entwicklersubstanz führen zu Haarfärbungen mit ausgezeichneten Echtheitseigenschaften, insbesondere was die Licht-, Wasch- und Reibechtheit anbetrifft, und lassen sich mit Reduktionsmitteln wieder abziehen.

Von besonderer Bedeutung ist weiterhin der durch die Verwendung der 4-Amino-2-aminomethylphenole in den Haarfärbemitteln gemäß vorliegender Anmeldung in toxikologischer und dermatologischer Hinsicht erzielte Fortschritt im Vergleich zu bekannten, für die Erzielung von Rottönen gebräuchlichen Entwicklersubstanzen.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre gute Farbintensität aus.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

In den nachstehenden Beispielen soll der Gegenstand der Erfindung näher erläutert werden, ohne diesen hierauf zu beschränken.

## Herstellungsbeispiele

### Beispiel 1 2-Acetamidomethyl-4-amino-phenol

(Verfahren A)

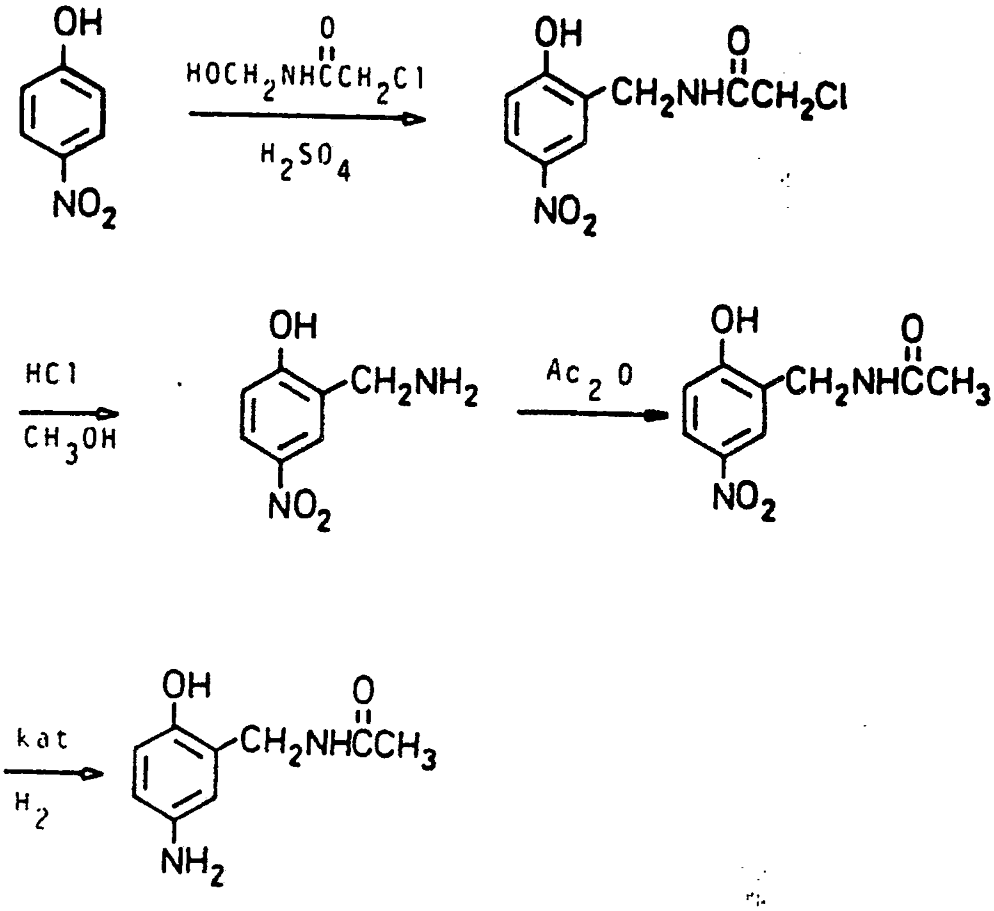


Stufe 1: 2-Chloracetamidomethyl-4-nitro-phenol

13,9 g (0,1 mol) 4-Nitro-phenol und 12,35 g (0,13 mol) N-Hydroxymethyl-chloracetamid (analog hergestellt nach der Vorschrift von J. D. Milkowski, D. F. Veber und R. Hirschmann, Org. Synth. 59 , Seite 190 bis 195 (1979)) werden zusammen in einer Reibschale fein verrieben und portionsweise unter Rühren in 100 ml konzentrierter Schwefelsäure eingetragen. Dabei erhöht sich die Temperatur auf 53° C. Man läßt über Nacht bei Raumtemperatur rühren und gießt dann den Reaktionsansatz auf Eis. Vom ausgefallenen Niederschlag wird abgesaugt. Der Rückstand kann ohne weitere Reinigung in Stufe 2 eingesetzt werden.

Die trockene Verbindung schmilzt bei über 230° C unter Zersetzung.

Ausbeute: 22 g (89,9 Prozent der Theorie)

Stufe 2: 2-Aminomethyl-4-nitro-phenol

12,2 g (0,056 mol) der Chloracetamidoverbindung der Stufe 1 werden in 100 ml Ethanol und 30 ml konzentrierter Salzsäure 4 Stunden lang unter Rückfluß erhitzt. Nach dem Verdünnen mit Wasser wird mit Natriumbicarbonat neutralisiert und vom ausgefallenen Niederschlag unter Nachwaschen mit Wasser abgesaugt. Der Rückstand wird durch Umfällen gereinigt. Hierzu werden 3 g der Substanz heiß in 25 ml Wasser/5 ml Eisessig gelöst. Nach dem Abkühlen wird filtriert und das Filtrat mit Ammoniak neutralisiert.

Die reine Aminomethylverbindung fällt aus, sie wird durch Absaugen isoliert und mit Wasser und Isopropanol gewaschen.
Ausbeute: 6,2 g (66,2 Prozent der Theorie)

Stufe 3: 2-Acetamidomethyl-4-nitro-phenol

2 g (0,012 mol) der Aminomethylverbindung aus Stufe 2 werden in 20 ml Eisessig gelöst und mit 10 ml (10,87 g = 0,11 mol) Acetanhydrid über Nacht bei Raumtemperatur gerührt.
Anschließend werden der Eisessig und das überschüssige Acetanhydrid im Vakuum abdestilliert, und der Rückstand wird aus Butanol umkristallisiert. Man erhält die Monoacetylverbindung in Form von gelblichen Kristallen vom Schmelzpunkt 187° C.
Ausbeute: 2,3 g (90,4 Prozent der Theorie)

**Elementaranalyse:**

| | berechnet | gefunden |
|---|---|---|
| % C | 51,42 | 51,90 |
| % H | 4,79 | 4,84 |

NMR-Spektrum:

11,26 (s, breit, OH, das Signal verschwindet beim Schütteln der Probe mit $D_2O$), 8,42 (t, breit, J = 6 Hz, NH, das Signal verschwindet beim Schütteln der Probe mit $D_2O$), 8,09 und 8,02 (Signal nicht aufgelöst, 3-H und 5-H), 6,99 (d, J = 10 Hz, 6-H), 4,24 (d, J = 6 Hz, Benzyl-$CH_2$, beim Schütteln der Probe mit $D_2O$ verschwindet die Kopplung), 1,92

(s, $\overset{\overset{O}{\|}}{C}CH_3$).

IR-Spektrum: (KBr-Preßling).

646, 762, 848, 918, 1021, 1055, 1093, 1145, 1248, 1298, (breit), 1345, 1495, 1540 (Schulter), 1590 (breit), 1630 (Schulter), 2000 bis 3500 (breit, scharfe Bande bei 3380) nm.

Stufe 4: 2-Acetamidomethyl-4-amino-phenol

2,3 g (0,012 mol) der Nitroverbindung der Stufe 3 werden in Ethanol gelöst und an einem Platinkatalysator mit Wasserstoff hydriert. Nach Aufnahme der theoretischen Menge Wasserstoff wird vom Katalysator abfiltriert, mit Salzsäure angesäuert und das Lösungsmittel im Vakuum verdampft. Die Verbindung bildet ein nicht kristallisierendes Öl.
Ausbeute: 1,8 g (90,3 Prozent der Theorie)

Beispiel 2 N-(5-Amino-2-hydroxy-benzyl)-N',N'-diethyl-ethylendiamin

(Verfahren C)

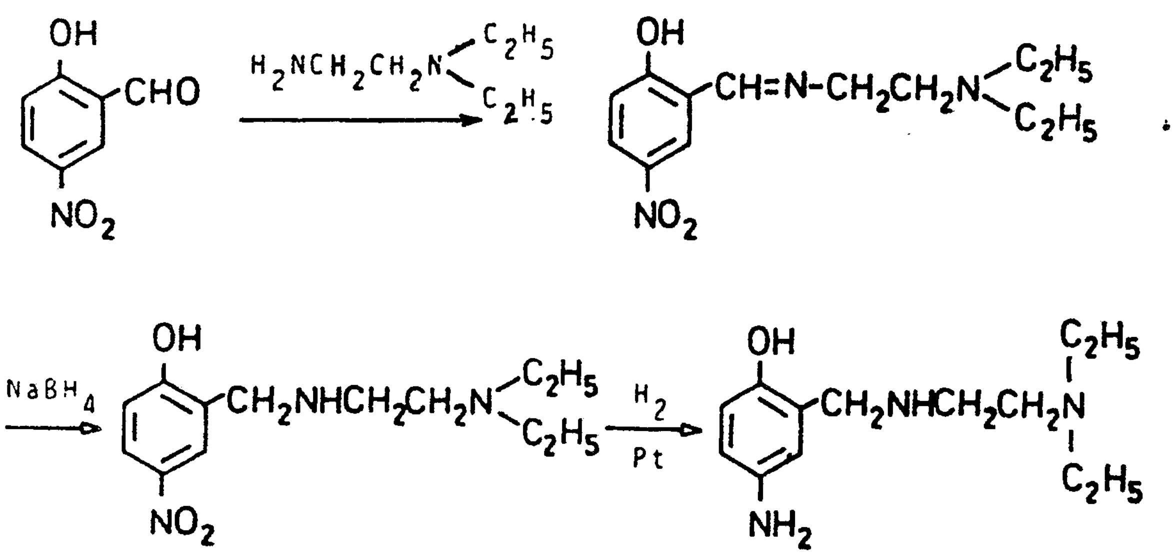


Stufe 1: N-(2-Hydroxy-5-nitro-benzyliden)-N',N'-diethyl-ethylendiamin

16,7 g (0,1 mol) 2-Hydroxy-5-nitro-benzaldehyd werden in 100 ml absolutem Methanol mit 20 g (0,17 mol) N,N-Diethyl-ethylendiamin 2 Stunden lang unter Rückfluß gekocht. Beim Abkühlen kristallisiert die Schiff'sche Base aus. Der Schmelzpunkt beträgt 116 bis 117° C. Die Verbindung wird ohne weitere Reinigung in Stufe 2 eingesetzt.

Ausbeute: 17,8 g (67 Prozent der Theorie)

Stufe 2: N-(2-Hydroxy-5-nitro-benzyl)-N',N'-diethyl-ethylendiamin

17,3 g (0,065 mol) Benzylidenverbindung der Stufe 1 werden in 100 ml Methanol gelöst und portionsweise unter Rühren bei Raumtemperatur mit insgesamt 3 g (0,079 mol) Natriumborhydrid versetzt. Nach 1 Stunde wird das Lösungsmittel im Vakuum abgedampft, der Rückstand mit konzentrierter Salzsäure angesäuert und wiederum zur Trockene eingedampft. Nach Zusatz von Aceton wird das Hydrochlorid durch Absaugen isoliert und aus Ethanol umkristallisiert. Durch Zusatz von Ammoniak wird die freie Base hergestellt, diese läßt sich durch Umkristallisation aus Ethylacetat reinigen.

Schmelzpunkt: 72° C.
Ausbeute: 8,5 g (49 Prozent der Theorie)

NMR-Spektrum:

8,01 (d, J = 3 Hz, 6'-H), 7,90 (dd, $J_1$ = 3 Hz, $J_2$ = 9 Hz, 4'-H), 6,82 (s, breit, OH, NH; das Signal verschwindet beim Schütteln der Probe mit $D_2O$), 6,37 (d, J = 9 Hz, 3'-H), 3,97 (s, Benzyl-$CH_2$), 2,9 bis 2,45 (m, -N-$CH_2$-), 0,98 (t, J = 7 Hz, $CH_2CH_3$).

Stufe 3: N-(5-Amino-2-hydroxy-benzyl)-N',N'-diethyl-ethylendiamin

8,5 g (0,032 mol) der Nitroverbindung der Stufe 2 werden analog Beispiel 1, Stufe 4 mit Wasserstoff an einem Platinkatalysator hydriert. Nach der Aufarbeitung erhält man das sehr hygroskopische Dihydrochlorid.

Ausbeute: 4,9 g (65 Prozent der Theorie)

NMR-Spektrum:

11,4 bis 8,5 (breit, NH, OH; beim Schütteln der Probe mit $D_2O$ verschwinden die Signale), 7,46 (s, 6'-H, Signal durch Kopplung mit 4-H verbreitert), 7,32 und 7,12 (d, J = 9 Hz, 3'-H und 4'-H, Signale jeweils verbreitert), 4,3 bis 3,0 (m, $-N-CH_2-$), 1.25 (t, J = 7 Hz, $CH_2-CH_3$).

Beispiel 3 N-(5'-Amino-2'-hydroxybenzyl)-oxazolidin-2-on

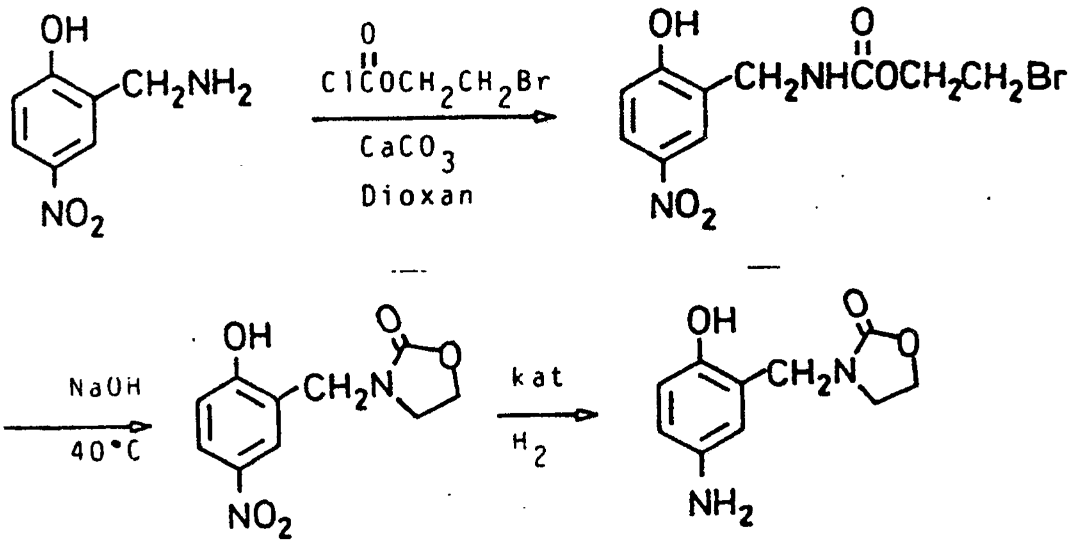


Stufe 1: N-(2'-Hydroxy-5'-nitrobenzyl)-carbamidsäure-2-bromethylester

16.8 g 2-Aminomethyl-4-nitrophenol (Beispiel 1, Stufe 2) werden in 100 ml Dioxan mit 8 g Calciumcarbonat aufgeschlämmt und auf 95° C erwärmt. Unter Rühren läßt man 20 g Chlorameisensäurebromethylester zutropfen und erwärmt anschließend noch 2 h auf die gleiche Temperatur. Man gießt auf Eiswasser, zersetzt mit konz. Salzsäure überschüssiges Calciumcarbonat und saugt unter Nachwaschen mit Wasser vom abgeschiedenen Urethan ab. Man erhält hellgelbe Kristalle vom Schmelzpunkt 128° C.

Stufe 2: N-(2'-Hydroxy-5'-nitrobenzyl)-oxazolidin-2-on

6 g des Urethans aus Stufe 1 werden in 270 ml 1 %-iger Natronlauge 1 h auf 40° C erwärmt. Beim Ansäuern mit Essigsäure fällt das Oxazolidinon aus. Nach Umkristallisieren aus n-Butanol schmilzt die Verbindung bei 228° C.

IR-Spektrum (KBr-Preßling):

752, 825, 1035, 1208, 1280, 1348, 1495, 1595, 1705 (intensiv), 3200 (breit) nm.

Stufe 3: N-(5'-Amino-2'-hydroxybenzyl)-oxazolidin-2-on

Reduktion der Nitroverbindung der Stufe 2 analog Beispiel 2, Stufe 2, ergibt die Aminoverbindung als freie Base vom Schmelzpunkt 165° C.

## BEISPIELE FÜR HAARFÄRBEMITTEL

### Beispiel 4 Haarfärbemittel in Gelform

| | |
|---|---|
| 0,50 g | 4-Amino-2-aminomethyl-phenol |
| 1,00 g | 2-Amino-4-[(2'-hydroxyethyl)-amino]-anisol-sulfat |
| 0,15 g | Natriumsulfit, wasserfrei |
| 5,00 g | Laurylalkohol-diglykolethersulfat-Natriumsalz (28-prozentige wäßrige Lösung) |
| 1,00 g | Hydroxyethylcellulose (hochviskos) |
| 10,00 g | Ammoniak (22-prozentige wäßrige Lösung) |
| 82,35 g | Wasser |
| 100,00 g | |

50 g des obigen Haarfärbemittels werden kurz vor dem Gebrauch mit 50 g Wasserstoffperoxidlösung (6-prozentig) vermischt, und das Gemisch wird anschließend auf blonde Naturhaare aufgetragen. Nach einer Einwirkungszeit von 30 Minuten bei 40° C wird mit Wasser gespült und getrocknet. Das Haar hat eine intensive, leuchtend rote Färbung mit einem leichten Violettstich erhalten.

### Beispiel 5 Haarfärbemittel in Gelform

| | |
|---|---|
| 0,35 g | 4-Amino-2-[(2'-hydroxyethyl)-aminomethyl]-phenol |
| 0,27 g | 5-Amino-2-methyl-phenol |
| 0,30 g | Ascorbinsäure |
| 15,00 g | Ölsäure |
| 7,00 g | Isopropanol |
| 10,00 g | Ammoniak (22-prozentige wäßrige Lösung) |
| 67,08 g | Wasser |
| 100,00 g | |

Man vermischt kurz vor dem Gebrauch 50 g dieses Haarfärbemittels mit 50 g Wasserstoffperoxidlösung (6-prozentig) und läßt das Gemisch 30 Minuten lang bei 40° C auf weißes menschliches Haar einwirken. Sodann wird mit Wasser gespült und getrocknet. Das Haar ist in einem orangen Farbton gefärbt.

Beispiel 6 Haarfärbemittel in Cremeform

| | |
|---|---|
| 1,00 g | 4-Amino-2-[bis-(2'-hydroxyethyl)-amino-methyl]-phenol |
| 1,10 g | 1-Naphthol |
| 15,00 g | Cetylalkohol |
| 0,30 g | Natriumsulfit, wasserfrei |
| 3,50 g | Laurylalkohol-diglykolethersulfat-Natrium-salz (28-prozentige wäßrige Lösung) |
| 3,00 g | Ammoniak (22-prozentige wäßrige Lösung) |
| 76,10 g | Wasser |
| 100,00 g | |

50 g dieses Haarfärbemittels werden kurz vor dem Gebrauch mit 50 g Wasserstoffperoxidlösung (6-prozentig) vermischt. Anschließend trägt man das Gemisch auf blonde Naturhaare auf und läßt es 30 Minuten lang bei 40° C einwirken. Danach wird mit Wasser gespült und getrocknet. Das Haar hat eine intensive Rotfärbung erhalten.

Beispiel 7 Haarfärbelösung

| | |
|---|---|
| 0,80 g | 4-Amino-2-dimethylaminomethyl-phenol |
| 0,12 g | Resorcin |
| 0,10 g | m-Aminophenol |
| 0,50 g | 5-Amino-2-methyl-phenol |
| 0,10 g | 2,4-Diamino-anisolsulfat |
| 0,05 g | 1-Naphthol |
| 10,00 g | Laurylalkohol-diglykolethersulfat-Natrium-salz (28-prozentige wäßrige Lösung) |
| 10,00 g | Ammoniak (22-prozentige wäßrige Lösung) |
| 78,33 g | Wasser |
| 100,00 g | |

Man vermischt kurz vor dem Gebrauch 50 g des vorstehenden Haarfärbemittels mit 50 g Wasserstoffperoxidlösung (6-prozentig) und läßt die Mischung 30 Minuten lang bei 40° C auf blonde Naturhaare einwirken. Sodann wird mit Wasser gespült und getrocknet. Das Haar ist in einem modischen goldorangen Farbton gefärbt.

Beispiel 8 Färbemittel in Gelform

| | |
|---|---|
| 0,90 g | 4-Amino-2-aminomethyl-phenol |
| 0,80 g | 2,5-Diamino-toluolsulfat |
| 0,15 g | 2,4-Diamino-anisolsulfat |
| 0,20 g | 5-Amino-2-methyl-phenol |
| 0,02 g | 1-[(2'-Ureidoethyl)-amino]-4-nitro-benzol |
| 0,05 g | 2-Nitro-p-phenylendiamin |
| 0,15 g | Natriumsulfit, wasserfrei |
| 2,50 g | Laurylalkohol-diglykolethersulfat-Natriumsalz (28-prozentige wäßrige Lösung) |
| 0,80 g | Hydroxyethylcellulose, hochviskos |
| 6,00 g | Ammoniak, (22-prozentige wäßrige Lösung) |
| 88,43 g | Wasser |
| 100,00 g | |

50 g des obigen Haarfärbemittels werden kurz vor dem Gebrauch mit 50 g Wasserstoffperoxidlösung (6-prozentig) gemischt. Die Mischung wird anschließend auf blonde Naturhaare aufgebracht. Nach einer Einwirkungszeit von 30 Minuten bei 40° C wird mit Wasser gespült und getrocknet. Das Haar hat eine modische braune Färbung erhalten.

Alle in der vorliegenden Anmeldung angegebenen Prozentzahlen stellen Gewichtsprozente dar.

## Ansprüche

1. Mittel zur oxidativen Färbung von Haaren auf der Basis einer Entwicklersubstanz-Kupplersubstanz-Kombination, dadurch gekennzeichnet, daß es als Entwicklersubstanz ein 4-Amino-2-aminomethyl-phenol der allgemeinen Formel (I)

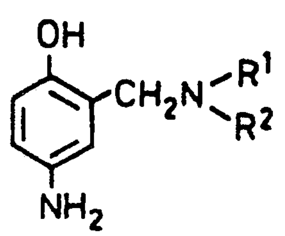

(I),

worin $R^1$ und $R^2$ unabhängig voneinander Wasserstoff, Alkyl, Hydroxyalkyl, Aminoalkyl, Mono- oder Dialkylaminoalkyl, Acylaminoalkyl, Aryl- oder Alkylsulfonylaminoalkyl, Carbamidoalkyl, Alkylsulfonyl - wobei der Alkylrest jeweils 1 bis 4 Kohlenstoffatome aufweist - Arylsulfonyl, Acyl oder Carbamoyl bedeuten oder durch $R^1$ und $R^2$ ein heterocyclischer, nicht aromatischer Fünf- oder Sechsring, der zusätzlich eine Oxo-Gruppe aufweisen kann, gebildet wird, oder dessen Salz enthält.

2. Mittel nach Anspruch 1, dadurch gekennzeichnet, daß die Entwicklersubstanz der Formel (I) ausgewählt ist aus 4-Amino-2-aminomethyl-phenol, 4-Amino-2-[(2'-hydroxyethyl)-aminomethyl]-phenol und 4-Amino-2-dimethylaminomethyl-phenol.

3. Mittel nach Anspruch 1 und 2, dadurch gekennzeichnet, daß die Kupplersubstanz ausgewählt ist aus 1-Naphthol, Resorcin, 4-Chlor-resorcin, 4,6-Dichlor-resorcin, 2-Methyl-resorcin, 2-Amino-4-[(2'-hydroxy ethyl)-amino]-anisol, 5-Amino-2-methyl-phenol, 2,4-Diamino-phenoxyethanol, 4-Amino-2-hydroxy-phenoxy-ethanol, m-Aminophenol, 3-Amino-2-methyl-phenol, 4-Hydroxy-1,2-methylendioxy-benzol, 4-Amino-1,2-methylendioxy-benzol, 4-[(2'-Hydroxyethyl-amino)]-1,2-methylendioxy-benzol, 2,4-Diamino-anisol, 2,4-Diamino-phenetol, 2,4-Diamino-benzylalkohol, m-Phenylendiamin, m-Toluylendiamin, 2,4-Diamino-phenylethyl-alkohol, 4-Hydroxy-indol, 3-Amino-5-hydroxy-2,6-dimethoxy-pyridin und 3,5-Diamino-2,6-dimethoxy-pyridin.

4. Mittel nach Anspruch 1 bis 3, dadurch gekennzeichnet, daß die Entwicklersubstanz der Formel (I) in einer Menge von 0,01 bis 3,0 Gewichtsprozent enthalten ist.

5. Mittel nach Anspruch 1 bis 4, dadurch gekennzeichnet, daß die Gesamtmenge der Entwickler-Kupplersubstanz-Kombination 0,1 bis 5,0 Gewichtsprozent beträgt.

6. Mittel nach Anspruch 1 bis 5, dadurch gekennzeichnet, daß es eine Farbkomponente enthält, welche ausgewählt ist aus 6-Amino-2-methyl-phenol, 2-Amino-5-methyl-phenol, Diamond Fuchsine (C.I. 42 510), Leather Ruby HF (C.I. 42 520), 2-Nitro-1,4-diamino- benzol, 2-Amino-4-nitro-phenol, 2-Amino-5-nitro-phenol, 2-Amino-4,6-dinitro-phenol, 2-Amino-5-[ (2'-hydroxyethyl )-amino]-nitrobenzol, 2-Methylamino-5-[bis-(2' -hydroxyethyl )-amino]-nitrobenzol, Acid Brown 4 (C.I. 14 805), 1 ,4-Diamino-anthrachinon und 1,4,5,8-Tetraamino-anthrachinon.

7. Mittel nach Anspruch 1 bis 6, dadurch gekennzeichnet, daß es Antioxidantien, vorzugsweise Asccrbinsäure, Thioglykolsäure oder Natriumsulfit enthält.

8. Mittel nach Anspruch 1 bis 7, dadurch gekennzeichnet, daß es einen pH-Wert zwischen 8,0 und 11,5 aufweist.

9. 4-Amino-2-aminomethyl-phenol der allgemeinen Formel (XII)

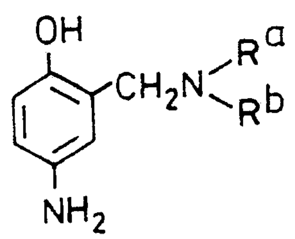

(XII),

wobei $R^a$ gleich Wasserstoff, Alkyl, Hydroxyalkyl, Aminoalkyl, Mono- oder Dialkylaminoalkyl, Acylaminoalkyl, Aryl - oder Alkylsulfonylaminoalkyl, Carbamoyl, Carbamidoalkyl, Acyl, Aryl- oder Alkylsulfonyl - wobei die Alkylreste jeweils 1 bis 4 Kohlenstoffatome aufweisen - bedeutet und $R^b$ gleich Aminoalkyl, Mono- oder Dialkylaminoalkyl, Acylaminoalkyl, Aryl- oder Alkylsulfonylaminoalkyl, Carbamoyl, Carbamidoalkyl, Acyl, Arylsulfonyl - wobei die Alkylreste jeweils 1 bis 4 Kohlenstoffatome aufweisen - ist.

10. 2-Acetamidomethyl-4-amino-phenol.

11. N-(5-Amino-2-hydroxy-benzyl)-N',N'-diethyl-ethylendiamin.

12. N-(5'-Amino-2'-hydroxy-benzyl)-oxazolidin-2-on

## Claims

1. Composition for the oxidative dyeing of hair, based on a developer substance/coupler substance combination, characterised in that as a developer substance it contains a 4-amino-2-aminomethyl-phenol of formula (I)

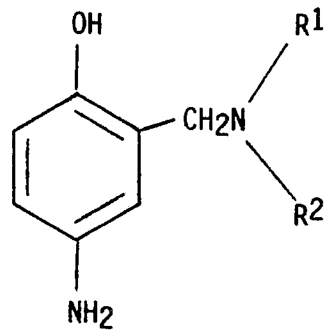

(I),

in which $R^1$ and $R^2$ independently from each other denote hydrogen, alkyl, hydroxyalkyl, aminoalkyl, mono- or dialkylaminoalkyl, carbamidoalkyl, alkylsulfonyl - the alkyl moiety containing in each case 1 to 4 carbon atoms - arylsulfonyl, acyl or carbamoyl, or in which $R^1$ and $R^2$ build a heterocyclic non-aromatic ring with 5 or 6 members, which additionally can contain an oxo-group, or its salt.

2. Composition according to claim 1, characterised in that the developer substance of formula (I) is selected from the group consisting of 4-amino-2-aminomethyl-phenol, 4-amino-2-[(2' hydroxyethyl)-aminomethyl]-phenol and 4-amino-2-dimethyl-aminomethyl-phenol.

3. Composition according to Claims 1 and 2, characterised in that the coupler substance is selected from the group consisting of 1-naphthol, resorcinol, 4-chloro resorcinol, 4,6-dichloro resorcinol, 2-methyl resorcinol, 2-amino-4-[(2'-hydroxyethyl)-amino]-anisole, 5-amino-2-methyl-phenol, 2,4-diamino-phenoxy ethanol, 4-amino-2-hydroxy-phenoxy ethanol], m-aminophenol, 3-amino-2-methyl phenol, 4-hydroxy-1,2-methylenedioxy benzene, 4-amino-1,2-methylenedioxy benzene, 4-[(2'-hydroxyethyl)-amino]-1,2-methylenedioxy benzene, 2,4-diamino anisole, 2,4-diamino phenetol, 2,4-diamino benzyl alcohol, m-phenylene diamine, m-tolylene diamine 2,4-diamino-phenylethyl alcohol, 4-hydroxy indol, 3-amino-5-hydroxy-2,6-dimethoxy pyridine and 3,5-diamino-2,6-dimethoxy pyridine.

4. Composition according to Claims 1 to 3, characterised in that it contains the developer substance of formula (I) in a quantity of 0,01 to 3,0 % by weight.

5. Composition according to Claims 1 to 4, characterised in that the total quantity of the developer substance/coupler substance combination is 0,1 to 5,0 % by weight.

6. Composition according to Claims 1 to 5, characterized in that it contains a dye component selected from the group consisting of 6-amino-2-methyl phenol, 2-amino-5-methyl phenol, Diamond Fuchsine (C.I. 42 510), Leather Ruby HF (C.I. 42 520), 2-nitro-1,4-diamino benzene, 2-amino-4-nitro phenol, 2-amino-5-nitro phenol, 2-amino-4,6-dinitro phenol, 2-amino-5-[(2'-hydroxyethyl)-amino]-nitro benzene, 2-methylamino-5-[bis(2'-hydroxyethyl)-amino]-nitro benzene, Acid Brown 4 (C.I. 14 805), 1,4-diamino anthraquione and 1,4,5,8-tetraamino anthraquinone.

7. Composition according to Claims 1 to 6, characterised in that it contains antioxidants, preferably ascorbic acid, thioglycolic acid or sodium sulphite.

8. Composition according to Claims 1 to 7, characterised in that it has a pH-value between 8.0 and 11.5.

9. 4-amino-2-aminomethyl phenol of formula (XII)

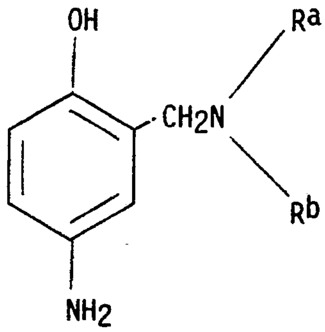

(XII),

in which $R^a$ denotes hydrogen, alkyl, hydroxyalkyl, aminoalkyl, mono- or dialkylaminoalkyl, acylaminoalkyl, aryl- or alkyl-sulfonylaminoalkyl, carbamoyl, carbamidoalkyl, acyl and aryl- or alkylsulfonyl - the alkyl moiety containing in each case 1 to 4 carbon atoms - and $R^b$ denotes aminoalkyl, mono- or dialkylaminoalkyl, acylaminoalkyl, aryl- or alkylsulfonylaminoalkyl, carbamoyl, carbamidoalkyl, acyl and arylsulfonyl - the alkyl moiety containing in each case 1 to 4 carbon atoms.

**10.** 2-acetamidomethyl-4-amino phenol.

**11.** N-(5-amino-2-hydroxy-benzyl)-N',N'-diethyl ethylene diamine.

**12.** N-(5'-amino-2'-hydroxy-benzyl)-oxazolidin-2-one.

## Revendications

**1.** Produit de coloration par oxydation des cheveux à base d'une combinaison développeur-copulateur, caractérisé en ce qu'il renferme, comme développeur, un 4-amino-2-aminométhyl-phénol répondant à la formule générale (I):

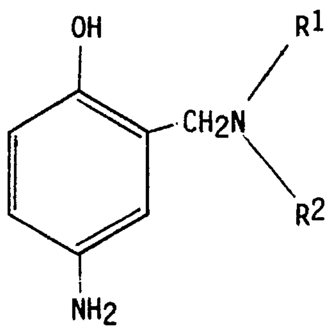

(I),

dans laquelle $R^1$ et $R^2$ représentent, indépendamment l'un de l'autre, l'hydrogène, un alkyle, un hydroxyalkyle, un aminoalkyle, un mono- ou dialkylaminoalkyle, un acylaminoalkyle, un aryl- ou alkylsulfonylamino-alkyle, un carbamidoalkyle, un alkylsufonyle (le reste alkyle comportant dans chaque cas 1 à 4 atomes de carbone), un arylsulfonyle, un acyle ou un carbamoyle, ou bien $R^1$ and $R^2$ forment un noyau pentagonal ou hexagonal non aromatique hétérocyclique, qui peut comporter en plus un groupe oxo, ou son sel.

**2.** Produit selon la revendication 1, caractérisé en ce que le développeur de la formule (I) est choisi parmi le 4-amino-2-amino-méthyl-phénol, le 4-amino-2-[(2'hydroxyéthyl)-aminométhyl]-phénol et le 4-amino-2-diméthyl-aminométhylphénol.

**3.** Produit selon les revendications 1 and 2, caractérisé en ce que le copulateur est choisi parmi le 1-naphtol, la résorcine, la 4-chloro-résorcine, la 4,6-dichloro-résorcine, la 2-méthyl-résorcine, le 2-amino-4-[(2'-hydroxyéthyl)amino]-anisole, le 5-amino-2-méthyl-phénol, le 2,4-diamino-phénoxyéthanol, le 4-amino-2-hydroxy-phénoxy-éthanol, le m-aminophénol, le 3-amino-2-méthyl-phénol, le 4-hydroxy-1,2-méthylènedioxy-benzène, le 4-amino-1,2-méthylènedioxy benzène, le 4-[(2-'hydroxyéthylamino)]-1,2-méthylènedioxy-benzène, le 2,4-diamino-anisole, le 2,4-diamino-phénétole, l'alcool 2,4-diamino-benzylique, la m-phénylène diamine, la m-toluylène-diamine, l'alcool 2,4-diamino-phényl-èthylique, le 4-hydroxy-indole, la 3-amino-5-hydroxy-2,6-di-méthoxy pyridine et la 3,5-diamino-2,6-diméthoxy-pyridine.

**4.** Produit selon les revendications 1 to 3, caractérisé en ce que le développeur répondant à la formule (I) est contenu dans une proportion de 0,01 à 3,0 % en poids.

**5.** Produit selon les revendications 1 à 4, caractérisé en ce que la proportion totale de la combinaison développeur-copulateur est de 0,1 à 5,0 % en poids.

**6.** Produit selon les revendications 1 à 5, caractérisé en ce qu'il renferme un composant colorant qui est choisi parmi le 6-amino-2-méthyl-phénol, le 2-amino-5-méthyl-phénol, Diamond Fuchsine (C.I. 42 510), Leather Ruby HF (C.I. 42 520), le 2-nitro-1,4-diamino-benzène, le 2-amino-4-nitro-phénol, le 2-amino-5-nitro-phénol, le 2-amino-4,6-dinitrophénol, le 2-amino-5-[(2'-hydroxy-éthyl)amino]-nitrobenzène, le 2-méthylamino-5-[bis(2'-hydroxy-éthyl)amino]-nitrobenzène, Acid Brown 4 (C.I. 14 805), la 1,4-diamino-anthraquinone et la 1,4,5,8-tétra-amino-anthraquinone.

**7.** Produit selon les revendications 1 à 6, caractérisé en ce qu'il renferme des anti-oxydantes de préférence de l'acid ascorbique, de l'acide thioglycolique ou du sulfite de sodium.

**8.** Produit selon les revendications 1 à 7, caractérisé en ce qu'il présente une valeur de pH comprise entre 8,0 et 11,5.

**9.** 4-amino-2-aminométhyl-phénol répondant à la formule générale (XII):

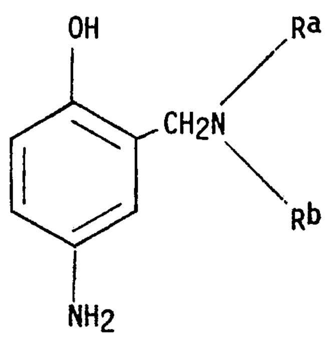

(XII),

dans laquelle $R^a$ représente l'hydrogène, un alkyle, un hydroxy-alkyle, un aminoalkyl, un mono- ou dialkylaminoalkyle, un acyl-aminoalkyl, aryl- ou alkylsulfonylaminoalkyl, un carbamoyle, un carbamidoalkyle, un acyle, un aryl- ou alkylsulfonyle (les restes alkyle comportant dans chaque cas 1 à 4 atomes de carbone), et $R^b$ représente un aminoalkyle, un mono- ou dialkylaminoalkyl, acyl-aminoalkyl, aryl- or alkylsulfonylaminoalkyle, un acylamino-alkyle, un aryl- ou alkyl-sulfonylaminoalkyle, un carbamoyle, un carbamidoalkyle, un acyle, un arylsulfonyle (les restes alkyle comportant chacun 1 à 4 atomes de carbone).

**10.** 2-acétamidométhyl-4-amino-phénol.

**11.** N-(5-amino-2-hydroxy-benzyl)-N,N'-diéthyl-éthylène-diamine.

**12.** N-(5'-amino-2'-hydroxy-benzyl)-oxazolidin-2-one.